# EUROPEAN PATENT SPECIFICATION

(11) **EP 0 878 468 B1**
(45) Date of publication and mention of the grant of the patent: **19.03.2003**
(21) Application number: 98303721.9
(22) Date of filing: 12.05.1998
(51) Int. Cl.: C07D 239/96

(54) **Process for producing 1-substituted tetrahydroquinazolines**
Verfahren zur Herstellung von 1-substituierten Tetrahydrochinazolinen
Procédé de production de tétrahydroquinazoline substituées en 1

(30) Priority: 13.05.1997 JP 12224297
(43) Date of publication of application: 18.11.1998
(73) Proprietor: Sumitomo Chemical Company, Limited, Chuo-ku Osaka 541-8550 (JP)
(72) Inventor: Asada, Sachiko, Takatsuki-shi, Osaka (JP); Komatsu, Masashi, Toyonaka-shi, Osaka (JP); Nishii, Shinji, Ibaraki-shi, Osaka (JP)
(74) Representative: Cresswell, Thomas Anthony

(56) References cited:
- EP-A- 0 775 697
- EP-A- 1 010 695
- PATENT ABSTRACTS OF JAPAN vol. 013, no. 205 (C-595), 15 May 1989 & JP 01 025767 A (FUJISAWA PHARMACEUT CO LTD), 27 January 1989,
- 'Handbook of Chemistry and Physics ed 1992-1993', CRC

## Description

The present invention relates to a process for producing 1-substituted tetrahydroquinazolines. More particularly, it relates to a process for producing 1-substituted tetrahydroquinazolines which comprises reacting tetrahydroquinazolines with hexamethyldisilazane; reacting the resultant product with a chloroalkanoate in the presence of an iodide of an alkali metal; followed by desilylation.

EP-A-0775697, which forms part of the state of the art by virtue of Article 54(3) EPC, also discloses a process for producing 1-substituted tetrahydroquinazolines which comprises reacting tetrahydroquinazolines with hexamethyldisilazane; reacting the resultant product with a chloroalkanoate in the presence of an iodide of an alkali metal; followed by desilylation.

EP-A-1010695, corresponding to WO-A-98/41514, which forms part of the state of the art by virtue of Article 54(3) EPC, discloses a process for producing certain 1-substituted tetrahydroquinazoline derivatives which comprises reacting the corresponding tetrahydroquinazolines with a silylating agent and then with a compound of formula Cl-A-R² wherein R² is carboxy or protected carboxy and A is lower alkylene.

1-Substituted tetrahydroquinazolines are compounds known as intermediates for antiphlogistic, remedies for diabetic complication; it is also known that 1-substituted tetrahydroquinazolines are produced by reacting tetrahydroquinazolines with hexamethyldisilazane; reacting the resultant product with a bromoalkanoate; followed by desilylation (see e.g. JP-A-64-25767).

However, this process had the problem that expensive bromoalkanoate is used.

On the other hand, a process for producing 1-substituted tetrahydroquinazolines in which a cheap chloroalkanoate is used in place of the bromoalkanoate has the problem that the yield is drastically lowered.

The present inventors have intensively studied about the process for producing 1-substituted tetrahydroquinazolines so as to solve above-mentioned drawbacks. As a result, it has been found, according to the present invention, that the desired product can be produced with high yield in an industrially advantageous manner, when the chloroalkanoate is used in place of the bromoalkanoate and the reaction is conducted in the presence of an iodide of an alkali metal.

Accordingly, the present invention provides a process for producing 1-substituted tetrahydroquinazolines represented by the following formula (III): 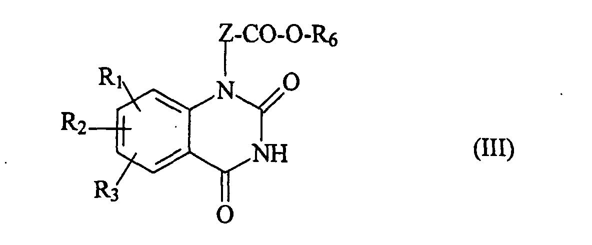 wherein
Z represents a methylene group which is optionally substituted by an alkyl group;
R₆ represents an alkyl group or an aralkyl group;
R₁ and R₂ independently represent a hydrogen atom, a halogen atom, a nitro group, an alkyl group which is optionally substituted with one or more halogen atoms, an alkenyl group which is optionally substituted with one or more halogen atoms, an aralkyl group which is optionally substituted with one or more halogen atoms, an alkoxy group which is optionally substituted with one or more halogen atoms, an alkoxycarbonyl group which is optionally substituted with one or more halogen atoms, an alkylcarbonyloxy group, an arylcarbonyloxy group, or an amino group represented by -XNR₄R₅
in which X represents a direct bond, an alkylene group or a carbonyl group, or N, R₄ and R₅ may form together a five- or six-membered heterocyclic ring which optionally contains another hetero atom which may be substituted or
when X is a direct bond or an alkylene group, R₄ and R₅ independently may additionally represent an alkyl group or
when X is a carbonyl group, R₄ and R₅ independently may additionally represent an alkylcarbonyloxy alkyl or arylcarbonyloxy alkyl group; and
R₃ represents a hydrogen atom, a halogen atom, a nitro group, an alkyl group which is optionally substituted with one or more halogen atoms, an alkenyl group which is optionally substituted with one or more halogen atoms, an aralkyl group which is optionally substituted with one or more halogen atoms, an alkoxy group which is optionally substituted with one or more halogen atoms, or an alkoxycarbonyl group which is optionally substituted with one or more halogen atoms which comprises reacting a tetrahydroquinazoline represented by the formula (I): 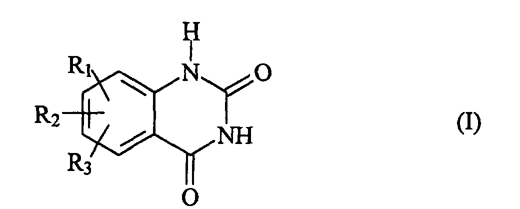 wherein R₁, R₂ and R₃ are as defined above, with hexamethyldisilazane in an aromatic hydrocarbon substituted with halogen; and reacting the resultant product with a chloroalkanoate represented by the formula (II):

Cl-Z-CO-O-R₆ (II)

wherein Z and R₆ are as defined above, in an aromatic hydrocarbon substituted with halogen in the presence of an iodide of an alkali metal, followed by desilylation.

The present invention also provides a process for producing a 1-substituted tetrahydroquinazoline of formula (III) which comprises reacting a tetrahydroquinazoline of formula (I) with hexamethyldisilazane; and reacting the resultant product with a chloroalkanoate of formula (II) in the presence of an iodide of an alkali metal while distilling off generated contents which have a lower boiling point, followed by desilylation.

Hereinafter, the present invention will be described in detail.

The substituents R₁ and R₂ in tetrahydroquinazolines (I), the starting material of the present invention, independently represent a hydrogen atom, a halogen atom, a nitro group, an alkyl group which is optionally substituted with one or more halogen atoms, an alkenyl group which is optionally substituted with one or more halogen atoms, an aralkyl group which is optionally substituted with one or more halogen atoms, an alkoxy group which is optionally substituted with one or more halogen atoms, an alkoxycarbonyl group which is optionally substituted with one or more halogen atoms, an alkylcarbonyloxy group, an arylcarbonyloxy group, or an amino group represented by -XNR₄R₅
in which X represents a direct bond, an alkylene group or a carbonyl group, and N, R₄ and R₅ may form together a five- or six-membered heterocyclic ring which optionally contains another hetero atom which may be substituted or
when X is a direct bond or an alkylene group, R₄ and R₅ independently may additionally represent an alkyl group or
when X is a carbonyl group, R₄ and R₅ independently may additionally represent an alkylcarbonyloxy alkyl or arylcarbonyloxy alkyl group.

Examples of the halogen atom include chlorine, bromine and fluorine.

Examples of the alkyl group which is optionally substituted with the halogen atom include lower alkyl groups such as methyl, ethyl, propyl, i-propyl, butyl, i-butyl, t-butyl, pentyl, i-pentyl and hexyl; monohalo lower alkyl groups such as chloromethyl, bromomethyl and chloropropyl; and dihalo lower alkyl groups such as 1,2-dichloroethyl, 1,2-dibromoethyl and 2,2-dichloroethyl; and trihalo lower alkyl groups such as trifluoromethyl.

Examples of the alkenyl group which is optionally substituted with the halogen atom include lower alkenyl groups such as 1-propenyl, 2-propenyl, 1-butenyl, 2-butenyl, 3-butenyl, 1-pentenyl, 2-pentenyl, 3-pentenyl and 4-pentenyl; monohalo lower alkenyl groups such as 3-chloro-1-propenyl and 3-chloro-1-butenyl; and dihalo lower alkenyl groups such as 3,4-dichloro-1-butenyl; and trihalo lower alkenyl groups such as 3,4,5-trichloro-1-pentenyl.

Examples of the aralkyl group which is optionally substituted with the halogen atom include benzyl, phenylethyl, 4-chlorobenzyl, 2,4-dichlorobenzyl and 2,4-dibromobenzyl.

Examples of the alkoxy group which is optionally substituted with the halogen atom include lower alkoxy groups such as methoxy, ethoxy, propoxy, i-propoxy, butoxy, i-butoxy, t-butoxy, pentyloxy, i-pentyloxy and hexyloxy; halogenated lower alkoxy groups such as chloromethoxy, bromomethoxy, 1-chloroe 2-chloroethoxy, 1-chloropropoxy, 2-chloropropoxy, 3-chloropropoxy, dichloromethoxy, dibromomethoxy, 1,2-dichloroethoxy, 2,2-dichloroethoxy, and trifluoromethoxy.

Examples of the alkoxycarbonyl group which is optionally substituted with the halogen atom include carbonyl groups substituted with an alkoxy group such as those exemplified above.

Examples of the alkylcarbonyloxy group include lower alkylcarbonyloxy groups such as acetoxy, propionyloxy, butylyloxy, i-butylyloxy, valeryloxy, i-valeryloxy and pivaloyloxy; and examples of the arylcarbonyloxy group include benzyloxy.

Examples of the alkylene group represented by X in the amino group -XNR₄R₅ include lower alkylene groups such as methylene, dimethylene, trimethylene and tetramethylene.

Examples of R₄ and R₅ as the lower alkyl group in the amino group -XNR₄R₅ include the same lower alkyl group as that exemplified above. In this case, specific examples of the amino group of -XNR₄R₅ include dimethylamino, diethylamino, dipropylamino and dibutylamino.

Specific examples of the amino group -XNR₄R₅, in case that N, R₄ and R₅ form together a five- or six-membered heterocyclic ring which optionally contains another heteroatom, include pyrrolyl, 2H,4H-pyrrolyl, pyrrolidino, pyrazolyl, piperidino, piperazinyl, morpholino and imidazolyl. t-butoxy, pentyloxy, i-pentyloxy and hexyloxy; halogenated lower alkoxy groups such as chloromethoxy, bromomethoxy, 1-chloroethoxy, 2-chloroethoxy, 1-chloropropoxy, 2-chloropropoxy, 3-chloropropoxy, dichloromethoxy, dibromomethoxy, 1,2-dichloroethoxy, 2,2-dichloroethoxy, and trifluoromethoxy.

Examples of the alkoxycarbonyl group which is optionally substituted with the halogen atom include carbonyl groups substituted with an alkoxy group such as those exemplified above.

Examples of the alkylcarbonyloxy group include lower alkylcarbonyloxy groups such as acetoxy, propionyloxy, butylyloxy, i-butylyloxy, valeryloxy, i-valeryloxy and pivaloyloxy; and examples of the arylcarbonyloxy group include benzyloxy.

Examples of the alkylene group represented by X in the amino group -XNR₄R₅ include lower alkylene groups such as methylene, dimethylene, trimethylene and tetramethylene.

Examples of R₄ and R₅ as the lower alkyl group in the amino group -XNR₄R₅ include the same lower alkyl group as that exemplified above. In this case, specific examples of the amino group -XNR₄R₅ include dimethylamino, diethylamino, dipropylamino and dibutylamino.

Specific examples of the amino group -XNR₄R₅, in case that N, R₄ and R₅ form together a five- or six-membered heterocyclic ring which optionally contains another hetero atom, include pyrrolyl, 2H,4H-pyrrolyl, pyrrolidino, pyrazolyl, piperidino, piperazinyl, morpholino and imidazolyl.

When the other hetero atom in the heterocyclic ring is a nitrogen atom, it may be substituted. Examples of the substituent include alkyl groups such as those exemplified above, aralkyl groups such as those exemplified above, aralkyl groups which are optionally substituted by an alkoxy group and a phenylcarbonyl group which is optionally substituted by an alkoxy group.

Examples of the tetrahydroquinazolines (I) include 2,4-dioxo-1,2,3,4-tetrahydroquinazoline, 5-chloro-2,4-dioxo-1,2,3,4-tetrahydroquinazoline, 6-chloro-2,4-dioxo-1,2,3,4-tetrahydroquinazoline, 7-chloro-2,4-dioxo-1,2,3,4-tetrahydroquinazoline, 8-chloro-2,4-dioxo-1,2,3,4-tetrahydroquinazoline, 5-bromo-2,4-dioxo-1,2,3,4-tetrahydroquinazoline, 6-bromo-2,4-dioxo-1,2,3,4-tetrahydroquinazoline, 7-bromo-2,4-dioxo-1,2,3,4-tetrahydroquinazoline, 8-bromo-2,4-dioxo-1,2,3,4-tetrahydroquinazoline, 5-fluoro-2,4-dioxo-1,2,3,4-tetrahydroquinazoline, 6-fluoro-2,4-dioxo-1,2,3,4-tetrahydroquinazoline, 7-fluoro-2,4-dioxo-1,2,3,4-tetrahydroquinazoline, 8-fluoro-2,4-dioxo-1,2,3,4-tetrahydroquinazoline, 5,6-dichloro-2,4-dioxo-1,2,3,4-tetrahydroquinazoline, 5,7-dichloro-2,4-dioxo-1,2,3,4-tetrahydroquinazoline, 5,8-dichloro-2,4-dioxo-1,2,3,4-tetrahydroquinazoline, 6,7-dichloro-2,4-dioxo-1,2,3,4-tetrahydroquinazoline, 6,8-dichloro-2,4-dioxo-1,2,3,4-tetrahydroquinazoline, 7,8-dichloro-2,4-dioxo-1,2,3,4-tetrahydroquinazoline, 5,6-dibromo-2,4-dioxo-1,2,3,4-tetrahydroquinazoline, 5,7-dibromo-2,4-dioxo-1,2,3,4-tetrahydroquinazoline, 5,8-dibromo-2,4-dioxo-1,2,3,4-tetrahydroquinazoline, 6,7-dibromo-2,4-dioxo-1,2,3,4-tetrahydroquinazoline, 6,8-dibromo-2,4-dioxo-1,2,3,4-tetrahydroquinazoline, 7,8-dibromo-2,4-dioxo-1,2,3,4-tetrahydroquinazoline, 5,6-difluoro-2,4-dioxo-1,2,3,4-tetrahydroquinazoline, 5,7-difluoro-2,4-dioxo-1,2,3,4-tetrahydroquinazoline, 5,8-difluoro-2,4-dioxo-1,2,3,4-tetrahydroquinazoline, 6,7-difluoro-2,4-dioxo-1,2,3,4-tetrahydroquinazoline, 7,8-difluoro-2,4-dioxo-1,2,3,4-tetrahydroquinazoline,
5-bromo-6-chloro-2,4-dioxo-1,2,3,4-tetrahydroquinazoline, 5-bromo-7-chloro-2,4-dioxo-1,2,3,4-tetrahydroquinazoline, 5-bromo-8-chloro-2,4-dioxo-1,2,3,4-tetrahydroquinazoline, 6-bromo-5-chloro-2,4-dioxo-1,2,3,4-tetrahydroquinazoline, 6-bromo-7-chloro-2,4-dioxo-1,2,3,4-tetrahydroquinazoline, 6-bromo-8-chloro-2,4-dioxo-1,2,3,4-tetrahydroquinazoline, 7-bromo-5-chloro-2,4-dioxo-1,2,3,4-tetrahydroquinazoline, 7-bromo-6-chloro-2,4-dioxo-1,2,3,4-tetrahydroquinazoline, 7-bromo-8-chloro-2,4-dioxo-1,2,3,4-tetrahydroquinazoline, 8-bromo-5-chloro-2,4-dioxo-1,2,3,4-tetrahydroquinazoline, 8-bromo-6-chloro-2,4-dioxo-1,2,3,4-tetrahydroquinazoline, 8-bromo-7-chloro-2,4-dioxo-1,2,3,4-tetrahydroquinazoline, 5,6,7-trichloro-2,4-dioxo-1,2,3,4-tetrahydroquinazoline, 5,6,8-trichloro-2,4-dioxo-1,2,3,4-tetrahydroquinazoline, 5,7,8-trichloro-2,4-dioxo-1,2,3,4-tetrahydroquinazoline, 6,7,8-trichloro-2,4-dioxo-1,2,3,4-tetrahydroquinazoline, 5,6,7-tribromo-2,4-dioxo-1,2,3,4-tetrahydroquinazoline, 5,6,8-tribromo-2,4-dioxo-1,2,3,4-tetrahydroquinazoline, 5,7,8-tribromo-2,4-dioxo-1,2,3,4-tetrahydroquinazoline, 6,7,8-tribromo-2,4-dioxo-1,2,3,4-tetrahydroquinazoline, 5,6,7-trifluoro-2,4-dioxo-1,2,3,4-tetrahydroquinazoline, 5,6,8-trifluoro-2,4-dioxo-1,2,3,4-tetrahydroquinazoline, 5,7,8-trifluoro-2,4-dioxo-1,2,3,4-tetrahydroquinazoline, 6,7,8-trifluoro-2,4-dioxo-1,2,3,4-tetrahydroquinazoline, 5-nitro-2,4-dioxo-1,2,3,4-tetrahydroquinazoline, 6-nitro-2,4-dioxo-1,2,3,4-tetrahydroquinazoline, 7-nitro-2,4-dioxo-1,2,3,4-tetrahydroquinazoline, 8-nitro-2,4-dioxo-1,2,3,4-tetrahydroquinazoline, 5-carboxy-2,4-dioxo-1,2,3,4-tetrahydroquinazoline, 6-carboxy-2,4-dioxo-1,2,3,4-tetrahydroquinazoline, 7-carboxy-2,4-dioxo-1,2,3,4-tetrahydroquinazoline, 8-carboxy-2,4-dioxo-1,2,3,4-tetrahydroquinazoline, 5-methyl-2,4-dioxo-1,2,3,4-tetrahydroquinazoline, 6-methyl-2,4-dioxo-1,2,3,4-tetrahydroquinazoline, 7-methyl-2,4-dioxo-1,2,3,4-tetrahydroquinazoline, 8-methyl-2,4-dioxo-1,2,3,4-tetrahydroquinazoline, 5-ethyl-2,4-dioxo-1,2,3,4-tetrahydroquinazoline, 6-ethyl-2,4-dioxo-1,2,3,4-tetrahydroquinazoline, 7-ethyl-2,4-dioxo-1,2,3,4-tetrahydroquinazoline, 8-ethyl-2,4-dioxo-1,2,3,4-tetrahydroquinazoline, 5-propyl-2,4-dioxo-1,2,3,4-tetrahydroquinazoline, 6-propyl-2,4-dioxo-1,2,3,4-tetrahydroquinazoline, 7-propyl-2,4-dioxo-1,2,3,4-tetrahydroquinazoline, 8-propyl-2,4-dioxo-1,2,3,4-tetrahydroquinazoline, 5-i-propyl-2,4-dioxo-1,2,3,4-tetrahydroquinazoline, 6-i-propyl-2,4-dioxo-1,2,3,4-tetrahydroquinazoline, 7-i-propyl-2,4-dioxo-1,2,3,4-tetrahydroquinazoline, 8-i-propyl-2,4-dioxo-1,2,3,4-tetrahydroquinazoline, 5-chloromethyl-2,4-dioxo-1,2,3,4-tetrahydroquinazoline, 6-chloromethyl-2,4-dioxo-1,2,3,4-tetrahydroquinazoline, 7-chloromethyl-2,4-dioxo-1,2,3,4-tetrahydroquinazoline, 8-chloromethyl-2,4-dioxo-1,2,3,4-tetrahydroquinazoline, 5-bromomethyl-2,4-dioxo-1,2,3,4-tetrahydroquinazoline, 6-bromomethyl-2,4-dioxo-1,2,3,4-tetrahydroquinazoline, 7-bromomethyl-2,4-dioxo-1,2,3,4-tetrahydroquinazoline, 8-bromomethyl-2,4-dioxo-1,2,3,4-tetrahydroquinazoline, 5-(1-chloroethyl)-2,4-dioxo-1,2,3,4-tetrahydroquinazoline, 6-(1-chloroethyl)-2,4-dioxo-1,2,3,4-tetrahydroquinazoline, 7-(1-chloroethyl)-2,4-dioxo-1,2,3,4-tetrahydroquinazoline, 8-(1-chloroethyl)-2,4-dioxo-1,2,3,4-tetrahydroquinazoline, 5-(2-chloroethyl)-2,4-dioxo-1,2,3,4-tetrahydroquinazoline, 6-(2-chloroethyl)-2,4-dioxo-1,2,3,4-tetrahydroquinazoline, 7-(2-chloroethyl)-2,4-dioxo-1,2,3,4-tetrahydroquinazoline, 8-(2-chloroethyl)-2,4-dioxo-1,2,3,4-tetrahydroquinazoline, 5-dichloromethyl-2,4-dioxo-1,2,3,4-tetrahydroquinazoline, 6-dichloromethyl-2,4-dioxo-1,2,3,4-tetrahydroquinazoline, 7-dichloromethyl-2,4-dioxo-1,2,3,4-tetrahydroquinazoline, 8-dichloromethyl-2,4-dioxo-1,2,3,4-tetrahydroquinazoline, 5-(1,2-dichloroethyl)-2,4-dioxo-1,2,3,4-tetrahydroquinazoline, 6-(1,2-dichloroethyl)-2,4-dioxo-1,2,3,4-tetrahydroquinazoline, 7-(1,2-dichloroethyl)-2,4-dioxo-1,2,3,4-tetrahydroquinazoline, 8-(1,2-dichloroethyl)-2,4-dioxo-1,2,3,4-tetrahydroquinazoline, 5-(2,2-dichloroethyl)-2,4-dioxo-1,2,3,4-tetrahydroquinazoline, 6-(2,2-dichloroethyl)-2,4-dioxo-1,2,3,4-tetrahydroquinazoline, 7-(2,2-dichloroethyl)-2,4-dioxo-1,2,3,4-tetrahydroquinazoline, 8-(2,2-dichloroethyl)-2,4-dioxo-1,2,3,4-tetrahydroquinazoline, 5-(2,2-dichloroethyl)-2,4-dioxo-1,2,3,4-tetrahydroquinazoline, 5-ethenyl-2,4-dioxo-1,2,3,4-tetrahydroquinazoline, 6-ethenyl-2,4-dioxo-1,2,3,4-tetrahydroquinazoline, 7-ethenyl-2,4-dioxo-1,2,3,4-tetrahydroquinazoline, 8-ethenyl-2,4-dioxo-1,2,3,4-tetrahydroquinazoline, 5-(1-propenyl)-2,4-dioxo-1,2,3,4-tetrahydroquinazoline, 6-(1-propenyl)-2,4-dioxo-1,2,3,4-tetrahydroquinazoline, 7-(1-propenyl)-2,4-dioxo-1,2,3,4-tetrahydroquinazoline, 8-(1-propenyl)-2,4-dioxo-1,2,3,4-tetrahydroquinazoline, 5-(2-propenyl)-2,4-dioxo-1,2,3,4-tetrahydroquinazoline, 6-(2-propenyl)-2,4-dioxo-1,2,3,4-tetrahydroquinazoline, 7-(2-propenyl)-2,4-dioxo-1,2,3,4-tetrahydroquinazoline, 8-(2-propenyl)-2,4-dioxo-1,2,3,4-tetrahydroquinazoline, 5-(1-butenyl)-2,4-dioxo-1,2,3,4-tetrahydroquinazoline, 6-(1-butenyl)-2,4-dioxo-1,2,3,4-tetrahydroquinazoline, 7-(1-butenyl)-2,4-dioxo-1,2,3,4-tetrahydroquinazoline, 8-(1-butenyl)-2,4-dioxo-1,2,3,4-tetrahydroquinazoline, , 5-(1-pentenyl)-2,4-dioxo-1,2,3,4-tetrahydroquinazoline, 6-(1-pentenyl)-2,4-dioxo-1,2,3,4-tetrahydroquinazoline, 7-(1-pentenyl)-2,4-dioxo-1,2,3,4-tetrahydroquinazoline, 8-(1-pentenyl)-2,4-dioxo-1,2,3,4-tetrahydroquinazoline, 5-(3-chloro-1-propenyl)-2,4-dioxo-1,2,3,4-tetrahydroquinazoline, 6-(3-chloro-1-propenyl)-2,4-dioxo-1,2,3,4-tetrahydroquinazoline, 7-(3-chloro-1-propenyl)-2,4-dioxo-1,2,3,4-tetrahydroquinazoline, 8-(3-chloro-1-propenyl)-2,4-dioxo-1,2,3,4-tetrahydroquinazoline, 5-(3,4-dichloro-1-butenyl)-2,4-dioxo-1,2,3,4-tetrahydroquinazoline, 6-(3,4-dichloro-1-butenyl)-2,4-dioxo-1,2,3,4-tetrahydroquinazoline, 7-(3,4-dichloro-1-butenyl)-2,4-dioxo-1,2,3,4-tetrahydroquinazoline, 8-(3,4-dichloro-1-butenyl)-2,4-dioxo-1,2,3,4-tetrahydroquinazoline, 5-methoxy-2,4-dioxo-1,2,3,4-tetrahydroquinazoline, 6-methoxy-2,4-dioxo-1,2,3,4-tetrahydroquinazoline, 7-methoxy-2,4-dioxo-1,2,3,4-tetrahydroquinazoline, 8-methoxy-2,4-dioxo-1,2,3,4-tetrahydroquinazoline, 5-ethoxy-2,4-dioxo-1,2,3,4-tetrahydroquinazoline, 6-ethoxy-2,4-dioxo-1,2,3,4-tetrahydroquinazoline, 7-ethoxy-2,4-dioxo-1,2,3,4-tetrahydroquinazoline, 8-ethoxy-2,4-dioxo-1,2,3,4-tetrahydroquinazoline, 5-propoxy-2,4-dioxo-1,2,3,4-tetrahydroquinazoline, 6-propoxy-2,4-dioxo-1,2,3,4-tetrahydroquinazoline, 7-propoxy-2,4-dioxo-1,2,3,4-tetrahydroquinazoline, 8-propoxy-2,4-dioxo-1,2,3,4-tetrahydroquinazoline, 5-i-propoxy-2,4-dioxo-1,2,3,4-tetrahydroquinazoline, 6-i-propoxy-2,4-dioxo-1,2,3,4-tetrahydroquinazoline, 7-i-propoxy-2,4-dioxo-1,2,3,4-tetrahydroquinazoline, 8-i-propoxy-2,4-dioxo-1,2,3,4-tetrahydroquinazoline, 5-t-butoxy-2,4-dioxo-1,2,3,4-tetrahydroquhnazoline, 6-t-butoxy-2,4-dioxo-1,2,3,4-tetrahydroquinazoline, 7-t-butoxy-2,4-dioxo-1,2,3,4-tetrahydroquinazoline, 8-t-butoxy-2,4-dioxo-1,2,3,4-tetrahydroquinazoline, 5-chloromethoxy-2,4-dioxo-1,2,3,4-tetrahydroquinazoline, 6-chloromethoxy-2,4-dioxo-1,2,3,4-tetrahydroquinazoline, 7-chloromethoxy-2,4-dioxo-1,2,3,4-tetrahydroquinazoline, 8-chloromethoxy-2,4-dioxo-1,2,3,4-tetrahydroquinazoline, 5-bromomethoxy-2,4-dioxo-1,2,3,4-tetrahydroquinazoline, 6-bromomethoxy-2,4-dioxo-1,2,3,4-tetrahydroquinazoline, 7-bromomethoxy-2,4-dioxo-1,2,3,4-tetrahydroquinazoline, 8-bromomethoxy-2,4-dioxo-1,2,3,4-tetrahydroquinazoline, 5-(2-chloroethoxy)-2,4-dioxo-1,2,3,4-tetrahydroquinazoline, 6-(2-chloroethoxy)-2,4-dioxo-1,2,3,4-tetrahydroquinazoline, 7-(2-chloroethoxy)-2,4-dioxo-1,2,3,4-tetrahydroquinazoline, 8-(2-chloroethoxy)-2,4-dioxo-1,2,3,4-tetrahydroquinazoline, 5-(1-chloropropoxy)-2,4-dioxo-1,2,3,4-tetrahydroquinazoline, 6-(1-chloropropoxy)-2,4-dioxo-1,2,3,4-tetrahydroquinazoline, 7-(1-chloropropoxy)-2,4-dioxo-1,2,3,4-tetrahydroquinazoline, 8-(1-chloropropoxy)-2,4-dioxo-1,2,3,4-tetrahydroquinazoline, 5-(2-chloropropoxy)-2,4-dioxo-2,2,3,4-tetrahydroquinazoline, 6-(2-chloropropoxy)-2,4-dioxo-1,2,3,4-tetrahydroquinazoline, 7-(2-chloropropoxy)-2,4-dioxo-1,2,3,4-tetrahydroquinazoline, 8-(2-chloropropoxy)-2,4-dioxo-1,2,3,4-tetrahydroquinazoline, 5-(3-chloropropoxy)-2,4-dioxo-1,2,3,4-tetrahydroquinazoline, 6-(3-chloropropoxy)-2,4-dioxo-1,2,3,4-tetrahydroquinazoline, 7-(3-chloropropoxy)-2,4-dioxo-1,2,3,4-tetrahydroquinazoline, 8-(3-chloropropoxy)-2,4-dioxo-1,2,3,4-tetrahydroquinazoline, 5-dichloromethoxy-2,4-dioxo-1,2,3,4-tetrahydroquinazoline, 6-dichloromethoxy-2,4-dioxo-1,2,3,4-tetrahydroquinazoline, 7-dichloromethoxy-2,4-dioxo-1,2,3,4-tetrahydroquinazoline, 8-dichloromethoxy-2,4-dioxo-1,2,3,4-tetrahydroquinazoline, 5-dibromomethoxy-2,4-dioxo-1,2,3,4-tetrahydroquinazoline, 6-dibromomethoxy-2,4-dioxo-1,2,3,4-tetrahydroquinazoline, 7-dibromomethoxy-2,4-dioxo-1,2,3,4-tetrahydroquinazoline, 8-dibromomethoxy-2,4-dioxo-1,2,3,4-tetrahydroquinazoline, 5-trifluororomethoxy-2,4-dioxo-1,2,3,4-tetrahydroquinazoline, 6-trifluoromethoxy-2,4-dioxo-1,2,3,4-tetrahydroquinazoline, 7-trifluoromethoxy-2,4-dioxo-1,2,3,4-tetrahydroquinazoline, 8-trifluoromethoxy-2,4-dioxo-1,2,3,4-tetrahydroquinazoline, 5-chloromethoxycarbonyl-2,4-dioxo-1,2,3,4-tetrahydroquinazoline, 6-chloromethoxycarbonyl-2,4-dioxo-1,2,3,4-tetrahydroquinazoline, 7-chloromethoxycarbonyl-2,4-dioxo-1,2,3,4-tetrahydroquinazoline, 8-chloromethoxycarbonyl-2,4-dioxo-1,2,3,4-tetrahydroquinazoline, 5-bromomethoxycarbonyl-2,4-dioxo-1,2,3,4-tetrahydroquinazoline, 6-bromomethoxycarbonyl-2,4-dioxo-1,2,3,4-tetrahydroquinazoline, 7-bromomethoxycarbonyl-2,4-dioxo-1,2,3,4-tetrahydroquinazoline, 8-bromomethoxycarbonyl-2,4-dioxo-1,2,3,4-tetrahydroquinazoline, 5-(1-chloroethoxycarbonyl)-2,4-dioxo-1,2,3,4-tetrahydroquinazoline, 6-(1-chloroethoxycarbonyl)-2,4-dioxo-1,2,3,4-tetrahydroquinazoline, 7-(1-chloroethoxycarbonyl)-2,4-dioxo-1,2,3,4-tetrahydroquinazoline, 8-(1-chloroethoxycarbonyl)-2,4-dioxo-1,2,3,4-tetrahydroquinazoline, 5-(2-chloroethoxycarbonyl)-2,4-dioxo-1,2,3,4-tetrahydroquinazoline, 6-(2-chloroethoxycarbonyl)-2,4-dioxo-1,2,3,4-tetrahydroquinazoline, 7-(2-chloroethoxycarbonyl)-2,4-dioxo-1,2,3,4-tetrahydroquinazoline, 8-(2-chloroethoxycarbonyl)-2,4-dioxo-1,2,3,4-tetrahydroquinazoline, 5-(1-chloropropoxycarbonyl)-2,4-dioxo-1,2,3,4-tetrahydroquinazoline, 6-(1-chloropropoxycarbonyl)-2,4-dioxo-1,2,3,4-tetrahydroquinazoline, 7-(1-chloropropoxycarbonyl)-2,4-dioxo-1,2,3,4-tetrahydroquinazoline, 8-(1-chloropropoxycarbonyl)-2,4-dioxo-1,2,3,4-tetrahydroquinazoline, 5-dichloromethoxycarbonyl-2,4-dioxo-1,2,3,4-tetrahydroquinazoline, 6-dichloromethoxycarbonyl-2,4-dioxo-1,2,3,4-tetrahydroquinazoline, 7-dichloromethoxycarbonyl-2,4-dioxo-1,2,3,4-tetrahydroquinazoline, 8-dichloromethoxycarbonyl-2,4-dioxo-1,2,3,4-tetrahydroquinazoline, 5-dibromomethoxycarbonyl-2,4-dioxo-1,2,3,4-tetrahydroquinazoline, 6-dibromomethoxycarbonyl-2,4-dioxo-1,2,3,4-tetrahydroquinazoline, 7-dibromomethoxycarbonyl-2,4-dioxo-1,2,3,4-tetrahydroquinazoline, 8-dibromomethoxycarbonyl-2,4-dioxo-1,2,3,4-tetrahydroquinazoline, 5-(1,2-dichloroethoxycarbonyl)-2,4-dioxo-1,2,3,4-tetrahydroquinazoline, 6-(1,2-dichloroethoxycarbonyl)-2,4-dioxo-1,2,3,4-tetrahydroquinazoline, 7-(1,2-dichloroethoxycarbonyl)-2,4-dioxo-1,2,3,4-tetrahydroquinazoline, 8-(1,2-dichloroethoxycarbonyl)-2,4-dioxo-1,2,3,4-tetrahydroquinazoline, 5-(2,2-dichloroethoxycarbonyl)-2,4-dioxo-1,2,3,4-tetrahydroquinazoline, 6-(2,2-dichloroethoxycarbonyl)-2,4-dioxo-1,2,3,4-tetrahydroquinazoline, 7-(2,2-dichloroethoxycarbonyl)-2,4-dioxo-1,2,3,4-tetrahydroquinazoline, 8-(2,2-dichloroethoxycarbonyl)-2,4-dioxo-1,2,3,4-tetrahydroquinazoline, 5-trifluoromethoxycarbonyl-2,4-dioxo-1,2,3,4-tetrahydroquinazoline, 6-trifluoromethoxycarbonyl-2,4-dioxo-1,2,3,4-tetrahydroquinazoline, 7-trifluoromethoxycarbonyl-2,4-dioxo-1,2,3,4-tetrahydroquinazoline, 8-trifluoromethoxycarbonyl-2,4-dioxo-1,2,3,4-tetrahydroquinazoline, 7,8-dimethyl-2,4-dioxo-1,2,3,4-tetrahydroquinazoline, 7,8-diethyl-2,4-dioxo-1,2,3,4-tetrahydroquinazoline, 6-benzyl-2,4-dioxo-1,2,3,4-tetrahydroquinazoline, 6-(2-phenylethyl)-2,4-dioxo-1,2,3,4-tetrahydroquinazoline, 6-(4-chlorobenzyl)-2,4-dioxo-1,2,3,4-tetrahydroquinazoline, 6-(2,4-dichlorobenzyl)-2,4-dioxo-1,2,3,4-tetrahydroquinazoline, 6-(2,4-dibromobenzyl)-2,4-dioxo-1,2,3,4-tetrahydroquinazoline, 5,6-dimethoxy-2,4-dioxo-1,2,3,4-tetrahydroquinazoline, 6,8-dimethoxy-2,4-dioxo-1,2,3,4-tetrahydroquinazoline, 5,8-dipropoxy-2,4-dioxo-1,2,3,4-tetrahydroquinazoline, 6-(N,N-dimethylamino)-2,4-dioxo-1,2,3,4-tetrahydroquinazoline, 6-(N,N-diethylamino)-2,4-dioxo-1,2,3,4-tetrahydroquinazoline, 6-(N,N-dimethylamino)-2,4-dioxo-1,2,3,4-tetrahydroquinazoline, 6-(N,N-dibutylamino)-2,4-dioxo-1,2,3,4-tetrahydroquinazoline, 6-(1-pyrrolyl)-2,4-dioxo-1,2,3,4-tetrahydroquinazoline, 6-(1-imidazolyl)-2,4-dioxo-1,2,3,4-tetrahydroquinazoline, 6-(1-pyrazolyl)-2,4-dioxo-1,2,3,4-tetrahydroquinazoline, 6-(2H,4H-pyrrolyl)-2,4-dioxo-1,2,3,4-tetrahydroquinazoline, 6-piperidino-2,4-dioxo-1,2,3,4-tetrahydroquinazoline, 6-morpholino-2,4-dioxo-1,2,3,4-tetrahydroquinazoline, 6-(4-methylpiperazinyl)-2,4-dioxo-1,2,3,4-tetrahydroquinazoline, 6-(4-chloromethylpiperazinyl)-2,4-dioxo-1,2,3,4-tetrahydroquinazoline, 6-(4-benzylpiperazinyl)-2,4-dioxo-1,2,3,4-tetrahydroquinazoline, 6-(4-(3-methoxybenzyl) piperazinyl)-2,4-dioxo-1,2,3,4-tetrahydroquinazoline, 6-(4-(phenylcarbonyl) piperazinyl)-2,4-dioxo-1,2,3,4-tetrahydroquinazoline, 6-(4-(3,4-dimethoxyphenylcarbonyl) piperazinyl)-2,4-dioxo-1,2,3,4-tetrahydroquinazoline, 6-(1-pyrrolylmethyl)-2,4-dioxo-1,2,3,4-tetrahydroquinazoline, 6-morpholinomethyl-2,4-dioxo-1,2,3,4-tetrahydroquinazoline, 7-(4-piperazinylmethyl)-2,4-dioxo-1,2,3,4-tetrahydroquinazoline, 6-(4-(3-phenylcarbonylpropy piperazinylcarbonyl)-2,4-dioxo-1,2,3,4-tetrahydroquinazoline, 7-(4-methylpiperazinylcarbonyl)-2,4-dioxo-1,2,3,4-tetrahydroquinazoline, 6-(4-benzylpiperidinocarbonyl)-2,4-dioxo-1,2,3,4-tetrahydroquinazoline, 6-acetoxy-2,4-dioxo-1,2,3,4-tetrahydroquinazoline, 6-propionyloxy-2,4-dioxo-1,2,3,4-tetrahydroquinazoline, 6-butylyloxy-2,4-dioxo-1,2,3,4-tetrahydroquinazoline, 6-i-butylyloxy-2,4-dioxo-1,2,3,4-tetrahydroquinazoline, 6-valeryloxy-2,4-dioxo-1,2,3,4-tetrahydroquinazoline, 6-pivaloyloxy-2,4-dioxo-1,2,3,4-tetrahydroquinazoline, 6-benzoyloxy-2,4-dioxo-1,2,3,4-tetrahydroquinazoline, 5,7-dimethyl-6-propionyloxy-2,4-dioxo-1,2,3,4-tetrahydroquinazoline and 8-chloro-5,6-dimethoxy-2,4-dioxo-1,2,3,4-tetrahydroquinazoline

The hexamethyldisilazane as a silylation agent is normally used in a 0.5 to 10 fold molar amount, preferably from about 1 to 5 fold molar amount, relative to the tetrahydro quinazolines (I).

The silylation reaction is normally carried out in a solvent at from room temperature to the reflux temperature of the solvent. The reaction can be accelerated by carrying it out in the presence of a salt such as ammonium sulfate, ammonium chloride, guanidine hydrochloride or pyridine hydrochloride, or of a mineral acid such as sulfuric acid or hydrochloric acid. In this case, the salt, the mineral acid or the like is normally used in a 0.01 to 1 fold molar amount, preferably about 0.05 to 0.5 fold molar amount, relative to the tetrahydroquinazolines (I).

The silylation reaction is preferably carried out under a reduced pressure and/ or in an inert gas flow. By carrying out the reaction under a reduced pressure and/ or in an inert gas flow, the reaction time can be shorten and/or reduction of the amount of hexamethyldisilazane can be attained. Range of the reduced pressure does not have a definite limit, but the range is usually from about 750 mm Hg, a slightly reduced pressure, to a pressure where the reaction solvent boils at room temperature, preferably from about 600 mm Hg to a pressure where the reaction solvent boils at room temperature, and more preferably the reduced pressure is about the pressure where the reaction solvent boils at room temperature. The inert gas may be any one which does not disturb the reaction, and includes nitrogen, helium, argon and the like. The inert gas may be a mixture of two or more gases. The amount of flow of the inert gas is not limited. Usually, the more is the amount, the more effective. Considering economical reasons etc., usually about 5-1000 ml/min., preferably 10-500 ml/min., is adopted relative to 1 l of reaction vessel.

The solvent for the silylation reaction may be any one which does not inhibit the reaction. Examples thereof include aromatic hydrocarbon such as benzene, toluene and xylene, chlorobenzene, o-dichlorobenzene, m-dichlorobenzene and bromobenzene; hydrocarbon halides such as dichloromethane and chloroform; ether solvent such as tetrahydrofuran and dioxane; and a mixture thereof. The solvent is normally used in a 0.5 to 10 fold amount by weight, preferably about 1 to 5 fold amount by weight, relative to the tetrahydroquinazolines (I).

The resultant silylated product may be used in the following step after isolation from the reaction mass, or may be subjected to the following step as it is.

After silylation, the resultant product is reacted with the chloroalkanoate (II) in the presence of the iodide of the alkaline metal, followed by desilylation. The substituent Z in the chloroalkanoate ( II ) is a methylene group which is optionally substituted with an alkyl group and R₆ is an alkyl group or an aralkyl group. Examples of the alkyl group include a lower alkyl group as that exemplified above. Examples of the methylene group which is optionally substituted with an alkyl group include methylene and methylmethylene, preferablly methylene. Examples of the aralkyl group include aralkyl group whose aromatic ring is optionally substituted with a nitro group, such as benzyl, 4-nitrobenzyl, phenylethyl, benzhydryl and trityl.

As the chloroalkanoate (II), those wherein R₆ is a lower alkyl group are preferred. Examples include methyl chloroacetate, ethyl chloroacetate, propyl chloroacetate, t-butyl chloroacetate and ethyl 2-chloropropionate.

The chloroalkanoate (II) is normally used in a 1 to 5 fold molar amount relative to the tetrahydroquinazolines (I).

The present invention is characterized by reacting the above-described chloroalkanoate (II) in the presence of the iodide of the alkaline metal. Examples of the iodide of the alkaline metal include potassium iodide, sodium iodide and lithium iodide. Among them, potassium iodide and sodium iodide are preferably used.

The iodide of the alkali metal is normally used in a 0.001 to 1 fold molar amount, preferably from about 0.1 to 1 fold molar amount, relative to the tetrahydroquinazolines (I).

The solvent for the reaction with the chloroalkanoate (II) may be any one which does not inhibit the reaction. Examples thereof include aromatic hydrocarbon such as benzene, toluene and xylene, chlorobenzene, o-dichlorobenzene, m-dichlorobenzene and bromobenzene; hydrocarbon halides such as dichloromethane and chloroform; ether solvent such as tetrahydrofuran and dioxane; and a mixture thereof. Among them, aromatic hydrocarbons are preferred. Particularly, aromatic hydrocarbons substituted with halogen such as chlorobenzene, o-dichlorobenzene, m-dichlorobenzene and bromobenzene are preferred. By using aromatic hydrocarbons substituted with halogen as the reaction solvent, the productivity of facilities can be improved by shortening the reaction time. The solvent is normally used in a 0.5 to 10 fold amount by weight, preferably about 1 to 5 fold amount by weight, relative to the tetrahydroquinazolines (I) used in the reaction.

The reaction temperature with the chloroalkanoate (II) is normally from about 0°C to a boiling point of the solvent, preferably from about 80°C to a boiling point of the solvent. As progress of the reaction, contents which have a lower boiling point, such as trimethylchlorosilane, is generated. The reaction can be carried out while distilling off the contents which have a lower boiling point .

The desilylation can be carried out by, for example, adding water or an alcohol such as methanol, ethanol and i-propanol. These are normally used in a 2 to 30 fold molar amount, preferably from about 10 to 20 fold molar amount, relative to the tetrahydroquinazolines (I).

Thus, 1-substituted tetrahydroquinazolines (III), the objective product, are produced. When the objective product is deposited as a solid in the reaction mass, it can be removed from the reaction mass, for example by filtration. When the objective product is dissolved in the reaction mass, it can be removed from the reaction mass by, for example, distilling off the solvent, adding water, extracting with an organic solvent and distilling off the organic solvent.

The 1-substituted tetrahydroquinazolines (III) can also be removed in the form of a salt, for example, with an inorganic base, such as an alkali metal salt, alkaline earth metal salt and ammonium salt; salt with an organic base, such as organic amine salt; addition salt with an inorganic acid, such as hydrochloride, hydrobromide, sulfate and phosphate; and addition salt with an organic acid, such as formate, acetate, trifluoroacetate, maleate, tartrate, methanesulfonate, benzenesulfonate and toluenesulfonate according to known processes.

The resultant 1-substituted tetrahydroquinazolines (III) or salt thereof can also be further purified, for example, by recrystallization or various chromatographic treatments.

According to the present invention, the 1-substituted tetrahydroquinazolines (III) can be produced with high yield by reacting with the chloroalkanoate (II) in the presence of the iodide of the alkaline metal.

The following Examples further illustrate the present invention in detail.

### Reference Example 1

A mixture of 17.3 g of toluene, 10 g of 7-chloro-2,4-dioxo-1,2,3,4-tetrahydroquinazoline, 19.7 g of hexamethyldisilazane and 1 g of ammonium sulfate was refluxed for 2 hours with stirring, and then excess hexamethyldisilazane and toluene (26.3 g) were distilled off at 55°C under a reduced pressure of 2.7 to 4.0 kPa (20 to 30 mmHg).

To this were added 8.45 g of potassium iodide and 27.6 g of ethyl chloroacetate and, after stirring continuously at 110 to 120°C for 12 hours, 40 g of ethanol was added and the mixture was refluxed for 1 hour. After cooling to room temperature, the deposited crystals were filtered, washed with ethanol and water, and then dried to obtain 13.9 g of crystals.

The content of 2-(7-chloro-2,4-dioxo-1,2,3,4-tetrahydroquinazolin-1-yl)ethyl acetate in this was 100%. (yield 96.4%)

### Reference Example 2

According to the same manner as that described in Reference Example 1 except that the amount of hexamethyldisilazane was changed to 38.2 g, the time of reflux was changed to 5 hours, the amount of toluene distilled off was 40.8 g, the amount of potassium iodide was changed to 1.69 g and time of stirring was changed to 32 hours, the reaction was carried out to obtain 14.1 g of crystals.

The content of 2-(7-chloro-2,4-dioxo-1,2,3,4-tetrahydroquinazolin-1-yl)ethyl acetate in this was 95.5%. (yield 93.7%)

### Comparative Example 1

According to the same manner as that described in Reference Example 1 except that potassium iodide was not used, the reaction was carried out to obtain 11.5 g of crystals.

The content of 2-(7-chloro-2,4-dioxo-1,2,3,4-tetrahydroquinazolin-1-yl)ethyl acetate in this was 56.8%. (yield 45.7%)

### Example 1

A mixture of 17 g of o-dichlorobenzene, 10 g of 7-chloro-2,4-dioxo-1,2,3,4-tetrahydroquinazoline, 19 g of hexamethyldisilazane and 1 g of ammonium sulfate was stirred at 120 °C for 10 hours, and then excess hexamethyldisilazane was distilled off at 55°C under a reduced pressure of 6.7 kPa (50 mmHg).

To this were added 1.6 g of potassium iodide and 27 g of ethyl chloroacetate and the resultant mixture was stirred continuously at 130°C to carry out the reaction. Progress of the reaction was checked with a high-speed liquid chromatography. It took 16 hours that the remaining amount of 7-chloro-2,4-dioxo7-chloro-2,4-dioxo-1,2,3,4-tetrahydroquinazoline in the reaction mass became 1% or less. The yield of 2-(7-chloro-2,4-dioxo-1,2,3,4-tetrahydroquinazolin-1-yl)ethyl acetate was 95 %.

### Example 2

According to the same manner as that described in Example 1 except that 17 g of 0-dichlorobenzene was replaced with 17 g of chlorobenzene and the reaction temperature after adding ethyl chloroacetate was changed from 130 °C to 120 °C. It took 21 hours that the remaining amount of 7-chloro-2,4-dioxo-1,2,3,4-tetrahydroquinazoline in the reaction mass became 1% or less. The yield of 2-(7-chloro-2,4-dioxo-1,2,3,4-tetrahydroquinazolin-1-yl)ethyl acetate was 91 %.

### Example 3

According to the same manner as that described in Example 1 except that 17 g of 0-dichlorobenzene was replaced with 17 g of toluene, excess hexamethyldisilazane was distilled off at 25°C under a reduced pressure of 2.7 kPa (20 mmHg) and the reaction temperature after adding ethyl chloroacetate was changed from 130 °C to 120 °C. It took 57 hours that the remaining amount of 7-chloro-2,4-dioxo-1,2,3,4-tetrahydroquinazoline in the reaction mass became 1% or less. The yield of 2-(7-chloro-2,4-dioxo-1,2,3,4-tetrahydroquinazolin-1-yl)ethyl acetate was 90 %.

### Example 4

A mixture of 40 g of o-dichlorobenzene, 20 g of 7-chloro-2,4-dioxo-1,2,3,4-tetrahydroquinazoline, 30 g of hexamethyldisilazane and 2 g of ammonium sulfate was stirred at 150 °C for 3 hours, and then excess hexamethyldisilazane was distilled off at 55°C under a reduced pressure of 1.3 kPa (10 mmHg).

To this were added 3.4 g of potassium iodide and 37 g of ethy chloroacetate and the resultant mixture was stirred continuously at 130°C to carry out the reaction, while distilling off the lower boiling point content such as trimethylchlorosilane generated with progress of the reaction. It took 21 hours that the remaining amount of 7-chloro-2,4-dioxo-1,2,3,4-tetrahydroquinazoline in the reaction mass became 1% or less.

After cooling to room temperature, ethyl acetate and water was added to deposit crystals. The deposited crystals were filtered, washed with methanol and water, and then dried to obtain 27.4 g of crystals.

The content of 2-(7-chloro-2,4-dioxo-1,2,3,4-tetrahydroquinazolin-1-yl)ethyl acetate in this was 98%. (yield 94%)

### Example 5

According to the same manner as that described in Example 4 except that excess hexamethyldisilazane and the contents which have a lower boiling point such as trimethylchlorosilane were not distilled off. It took 25 hours that the remaining amount of 7-chloro-2,4-dioxo-1,2,3,4-tetrahydroquinazoline in the reaction mass became 1% or less.

Same after-treatment as that in Example 4 was carried out to obtain 25.7 g of crystals.

The content of 2-(7-chloro-2,4-dioxo-1,2,3,4-tetrahydroquinazolin-1-yl)ethyl acetate in this was 97%. (yield 88%)

### Example 6

A mixture of 82 g of o-dichlorobenzene, 20.4 g of 7-chloro-2,4-dioxo-1,2,3,4-tetrahydroquinazoline, 16.4 g of hexamethyldisilazane and 0.8 g of sulfuric acid was stirred at 140°C for 7 hours in a 300ml vessel, while flowing nitrogen gas at 20 ml/min. Then, excess hexamethyldisilazane and a part of o-dichlorobenzene were distilled off under a reduced pressure.

To this were added 2.9 g of sodium iodide and 17.7 g of ethyl chloroacetate and, after stirring continuously at 111-132°C for 30 hours, a mixture of ethyl acetate and methanol was added to deposit crystals. The deposited crystals were filtered, washed with methanol and water, and then dried to obtain 25.1 g of crystals.

The content of 2-(7-chloro-2,4-dioxo-1,2,3,4-tetrahydroquinazolin-1-yl)ethyl acetate in this was 99%. (yield 92%)

### Example 7

A mixture of 596 g of o-dichlorobenzene, 151 g of 7-chloro-2,4-dioxo-1,2,3,4-tetrahydroquinazoline, 134 g of hexamethyldisilazane and 6 g of sulfuric acid was stirred at 100-140°C for 12 hours under a reduced pressure of 48.0 - 26.7 kPa (360-200 mm Hg). Then, excess hexamethyldisilazane and a part of o-dichlorobenzene were distilled off under a reduced pressure.

To this were added 23 g of sodium iodide and 185 g of ethyl chloroacetate and, after stirring continuously at 135 °C for 24 hours, a mixture of ethyl acetate and methanol was added to deposit crystals. The deposited crystals were filtered, washed with methanol and water, and then dried to obtain 207.7 g of crystals.

The content of 2-(7-chloro-2,4-dioxo-1,2,3,4-tetrahydroquinazolin-1-yl)ethyl acetate in this was 97%. (yield 95%)

## Claims

1. A process for producing a 1-substituted tetrahydroquinazoline represented by the formula (III): 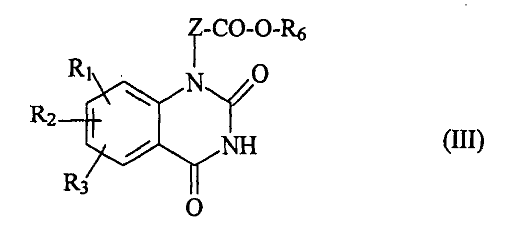 wherein
Z represents a methylene group which is optionally substituted by an alkyl group;
R₆ represents an alkyl group or an aralkyl group;
R₁ and R₂ independently represent a hydrogen atom, a halogen atom, a nitro group, an alkyl group which is optionally substituted with one or more halogen atoms, an alkenyl group which is optionally substituted with one or more halogen atoms, an aralkyl group which is optionally substituted with one or more halogen atoms, an alkoxy group which is optionally substituted with one or more halogen atoms, an alkoxycarbonyl group which is optionally substituted with one or more halogen atoms, an alkylcarbonyloxy group, an arylcarbonyloxy group, or an amino group represented by -XNR₄R₅
in which X represents a direct bond, an alkylene group or a carbonyl group, and N, R₄ and R₅ may form together a five- or six-membered heterocyclic ring which optionally contains another hetero atom which may be substituted or
when X is a direct bond or an alkylene group, R₄ and R₅ independently may additionally represent an alkyl group or
when X is a carbonyl group, R₄ and R₅ independently may additionally represent an alkylcarbonyloxy alkyl or arylcarbonyloxy alkyl group; and
R₃ represents a hydrogen atom, a halogen atom, a nitro group, an alkyl group which is optionally substituted with one or more halogen atoms, an alkenyl group which is optionally substituted with one or more halogen atoms, an aralkyl group which is optionally substituted with one or more halogen atoms, an alkoxy group which is optionally substituted with one or more halogen atoms, or an alkoxycarbonyl group which is optionally substituted with one or more halogen atoms which comprises reacting a tetrahydroquinazoline represented by the formula (I): 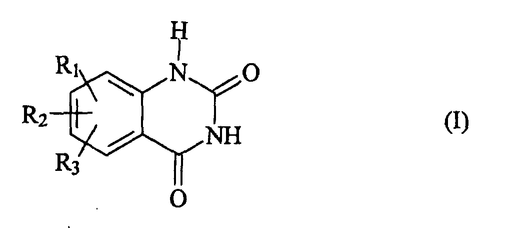 wherein R₁, R₂ and R₃ are as defined above, with hexamethyldisilazane in an aromatic hydrocarbon substituted with halogen; and
reacting the resultant product with a chloroalkanoate represented by the formula (II):
Cl-Z-CO-O-R₆ (II)
wherein Z and R₆ are as defined above, in an aromatic hydrocarbon substituted with halogen in the presence of an iodide of an alkali metal, followed by desilylation.

2. A process for producing a 1-substituted tetrahydroquinazoline of formula (III) as defined in claim 1, which comprises reacting a tetrahydroquinazoline of formula (I) as defined in claim 1 with hexamethyldisilazane; and reacting the resultant product with a chloroalkanoate of formula (II) as defined in claim 1 in the presence of an iodide of an alkali metal while distilling off generated contents which have a lower boiling point, followed by desilylation.

3. The process according to claim 1 or 2, wherein the substituent R₁ of the tetrahydroquinazoline (I) is a halogen atom and the substituent R₂ is a hydrogen atom.

4. The process according to any one of claims 1 to 3, wherein the tetrahydroquinazoline (I) is 7-chloro-2,4-dioxo-1,2,3,4-tetrahydroquinazoline.

5. The process according to any one of claims 1 to 4, wherein R₆ of the chloroalkanoate (II) is a lower alkyl group.

6. The process according to any one of claims 1 to 5, wherein Z of the chloroalkanoate (II) is methylene or methylmethylene.

7. The process according to any one of claims 1 to 6, wherein the chloroalkanoate (II) is ethyl chloroacetate.

8. The process according to any one of claims 1 to 7, wherein the chloroalkanoate (II) is used in a 1- to 5-fold molar amount relative to the tetrahydroquinazoline (I).

9. The process according to any one of claims 1 to 8, wherein the iodide of the alkali metal is potassium iodide or sodium iodide.

10. The process according to claim 2, which is carried out in at least one solvent selected from an aromatic hydrocarbon, a hydrocarbon halide and an ether solvent.

11. The process according to claim 2, wherein the solvent is an aromatic hydrocarbon substituted with halogen.

12. The process according to claim 1, wherein the reaction with the chloroalkanoate (II) is carried out while distilling off generated contents which have a lower boiling point.

## Patentansprüche

1. Verfahren zur Herstellung eines 1-substituierten Tetrahydrochinazolins gemäss der Formel (III): 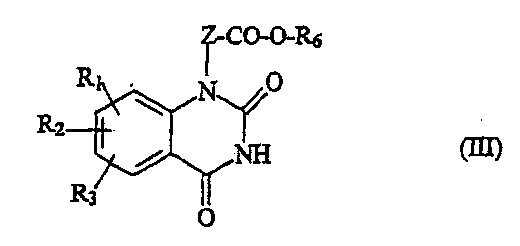 wobei
Z eine Methylengruppe bedeutet, die gegebenenfalls mit einer Alkylgruppe substituiert ist;
R₆ eine Alkylgruppe oder eine Aralkylgruppe bedeutet;
R₁ and R₂ unabhängig voneinander ein Wasserstoffatom, ein Halogenatom, eine Nitrogruppe, eine Alkylgruppe, die gegebenenfalls mit einem oder mehreren Halogenatomen substituiert ist, eine Alkenylgruppe, die gegebenenfalls mit einem oder mehreren Halogenatomen substituiert ist, eine Aralkylgruppe, die gegebenenfalls mit einem oder mehreren Halogenatomen substituiert ist, eine Alkoxygruppe, die gegebenenfalls mit einem oder mehreren Halogenatomen substituiert ist, eine Alkoxycarbonylgruppe, die gegebenenfalls mit einem oder mehreren Halogenatomen substituiert ist, eine Alkylcarbonyloxygruppe, eine Arylcarbonyloxygruppe oder eine Aminogruppe gemäss -XNR₄R₅ bedeuten, wobei
X eine direkte Bindung, eine Alkylengruppe oder eine Carbonylgruppe bedeutet, und
N, R₄ and R₅ miteinander einen heterocyclischen Fünfer- oder Sechserring bilden können, der gegebenenfalls ein weiteres Heteroatom enthält, das substituiert sein kann, oder wenn X eine direkte Bindung oder eine Alkylengruppe ist, können R₄ and R₅ unabhängig voneinander zusätztich eine Alkylgruppe bedeuten, oder
wenn X eine Carbonylgruppe ist, können R₄ and R₅ unabhängig voneinander zusätzlich eine Alkylcarbonyloxyalkylgruppe oder eine Arylcarbonyloxyalkylgruppe bedeuten; und
R₃ bedeutet ein Wasserstoffatom, ein Halogenatom, eine Nitrogruppe, eine Alkylgruppe, die gegebenenfalls mit einem oder mehreren Halogenatomen substituiert ist, eine Alkenylgruppe, die gegebenenfalls mit einem oder mehreren Halogenatomen substituiert ist, eine Aralkylgruppe, die gegebenenfalls mit einem oder mehreren Halogenatomen substituiert ist, eine Alkoxygruppe, die gegebenenfalls mit einem oder mehreren Halogenatomen substituiert ist, oder eine Alkoxycarbonylgruppe, die gegebenenfalls mit einem oder mehreren Halogenatomen substituiert ist, **dadurch gekennzeichnet, dass** man ein Tetrahydrochinazolin der Formel (I): 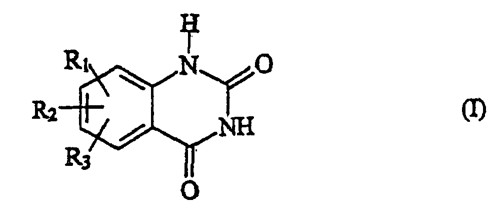 wobei R₁, R₂ and R₃ wie oben definiert sind, mit Hexamethyldisilazan in einem aromatischen, mit Halogen substituierten Kohlenwasserstoff zur Reaktion bringt, und das resultierende Produkt mit einem Chloroalkanoat der Formel (II)
Cl-Z-CO-O-R₆ (II)
wobei Z and R₆ wie oben definiert sind, in einem aromatischen, mit Halogen substituierten Kohlenwasserstoff in Gegenwart eines Alkaliiodides zur Reaktion bringt und anschliessend desilyliert.

2. Verfahren zur Herstellung eines 1-substituierten Tetrahydrochinazolins - gemäss der Formel (III) wie in Anspruch 1 definiert, **dadurch gekennzeichnet, dass** man ein Tetrahydrochinazolin - der Formel (I) wie in Anspruch 1 definiert - mit Hexamethyldisilazan zur Reaktion bringt und das resultierende Produkt mit einem Chloroalkanoat der Formel (II) wie in Anspruch 1 definiert - in Gegenwart eines Alkaliiodides zur Reaktion bringt, wobei gebildete Inhaltsstoffe, die einen niedrigeren Siedepunkt haben, abdestilliert werden und man anschliessend desilyliert.

3. Verfahren nach Anspruch 1 oder 2, **dadurch gekennzeichnet, dass** der Substituent R₁ des Tetrahydrochinazolins (I) ein Halogenatom und der Substituent R₂ ein Wasserstoffatom ist.

4. Verfahren nach einem der Ansprüche 1 bis 3, **dadurch gekennzeichnet, dass** das Tetrahydrochinazolin (I) 7-chloro-2,4-dioxo-1,2,3,4-Tetrahydrochinazolin ist.

5. Verfahren nach einem der Ansprüche 1 bis 4, **dadurch gekennzeichnet, dass** R₆ im Chloroalkanoat (II) eine niedrigere Alkylgruppe ist.

6. Verfahren nach einem der Ansprüche 1 bis 5, **dadurch gekennzeichnet, dass** Z im Chloroalkanoat (II) Methylen oder Methylmethylen ist.

7. Verfahren nach einem der Ansprüche 1 bis 6, **dadurch gekennzeichnet, dass** das Chloroalkanoat (II) Ethylchloroacetat ist.

8. Verfahren nach einem der Ansprüche 1 bis 7, **dadurch gekennzeichnet, dass** das Chloroalkanoat (II) in einer 1- bis 5-fach molaren Menge im Verhältnis zum Tetrahydrochinazolin (I) verwendet wird.

9. Verfahren nach einem der Ansprüche 1 bis 8, **dadurch gekennzeichnet, dass** das Alkaliiodid Kalium- oder Natriumiodid ist.

10. Verfahren nach Anspruch 2, **dadurch gekennzeichnet, dass** es in wenigstens einem Lösungsmittel durchgeführt wird, das aus einem aromatischen Kohlenwasserstoff, einem Halogenkohlenwasserstoff und einem Äther ausgewählt ist.

11. Verfahren nach Anspruch 2, **dadurch gekennzeichnet, dass** das Lösungsmittel ein aromatischer Halogenkohlenwasserstoff ist.

12. Verfahren nach Anspruch 1, **dadurch gekennzeichnet, dass** die Reaktion mit dem Chloroalkanoat (II) durchgeführt wird, während gebildete Inhaltsstoffe, die einen niedrigeren Siedepunkt haben, abdestilliert werden.

## Revendications

1. Procédé de production d'une tétrahydroquinazoline substituée en 1 représentée par la formule (III) : 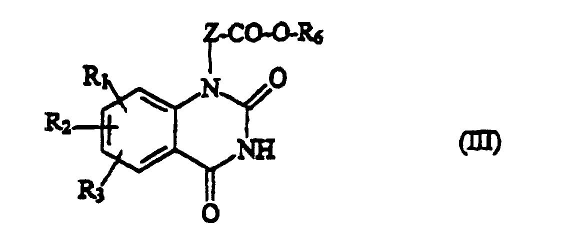 dans laquelle
Z représente un groupement de méthylène qui est facultativement substitué par un groupement alkyle,
R₆ représente un groupement alkyle ou un groupe aralkyle,
R₁ et R₂ représente indépendamment un atome d'hydrogène, un atome d'halogène, un groupe nitro, un groupement alkyle qui est facultativement substitué par un ou plusieurs atomes d'halogène, un groupement alcényle qui est facultativement substitué par un ou plusieurs atomes d'halogène, un groupement aralkyle qui est facultativement substitué par un ou plusieurs atomes d'halogène, un groupement alcoxy qui est facultativement substitué par un plusieurs atomes d'halogène, un groupement alcoxycarbonyle qui est facultativement substitué par un ou plusieurs atomes d'halogène, un groupement alkylcarbonyloxy, un groupement arylcarbonyloxy, ou un groupement amino représenté par -XNR₄R₅ dans lequel X représente une liaison directe, un groupement alkylène ou un groupement carbonyle, et N, R₄ et R₅ peuvent former ensemble un noyau hétérocyclique à cinq ou six chaînons qui contient facultativement un autre hétéro-atome qui peut être substitué ou
lorsque X est une liaison directe ou un groupement alkylène, R₄ et R₅ peuvent en outre représenter indépendamment un groupement alkyle ou
lorsque X est un groupement carbonyle, R₄ et R₅ peuvent en outre représenter indépendamment un groupement alkylcarbonyloxyalkyle ou arylcarbonyloxyalkyle, et
R₃ représente un atome d'hydrogène, un atome d'halogène, un groupement nitro, un groupement alkyle qui est facultativement substitué par un ou plusieurs atomes d'halogène, un groupement alcényle qui est facultativement substitué par un ou plusieurs atomes d'halogène, un groupement aralkyle qui est facultativement substitué par un ou plusieurs atomes d'halogène, un groupement alcoxy qui est facultativement substitué par un ou plusieurs atomes d'halogène, ou un groupement alcoxycarbonyle qui est facultativement substitué par un ou plusieurs atomes d'halogène
qui comprend la mise en réaction d'une tétrahydroquinazoline représentée par la formule (I) : 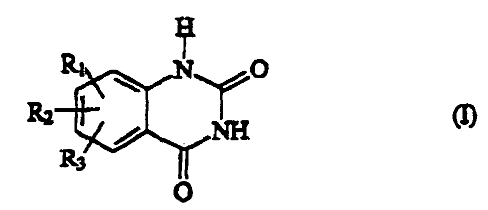 dans laquelle R₁, R₂ et R₃ sont tels que définis ci-dessus, avec de l'hexaméthyldisilazane dans un hydrocarbure aromatique substitué par un halogène, et
la mise en réaction du produit résultant avec un chloro-alkanoate représenté par la formule (II) :
Cl-Z-CO-O-R₆ (II)
dans laquelle Z et R₆ sont tels que définis ci-dessus, dans un hydrocarbure aromatique substitué par un halogène en présence d'un iodure d'un métal alcalin, suivi par une désilylation

2. Procédé de production d'une tétrahydroquinazoline substituée en 1 de formule (III) tels que définie dans la revendication 1, qui comprend la mise en réaction d'une tétrahydroquinazoline de formule (I) telle que définie dans la revendication 1 avec de l'hexaméthyldisilazane, et la mise en réaction du produit résultant avec un chloro-alkanoate de formule (II) tel que défini dans la revendication 1 en présence d'un iodure d'un métal alcalin tout en séparant par distillation les composés générés qui présentent un point d'ébullition inférieur, suivis par une désilylation.

3. Procédé selon la revendication 1 ou 2, dans lequel le substituant R₁ de la tétrahydroquinazoline (I) est un atome d'halogène et le substituant R₂ est un atome d'hydrogène.

4. Procédé selon l'une quelconque des revendications 1 à 3, dans lequel la tétrahydroquinazoline (I) est la 7-choro-2,4-dioxo-1,2,3,4-tétrahydroquinazoline.

5. Procédé selon l'une quelconque des revendications 1 à 4, dans lequel R₆ du chloro-alkanoate (II) est un groupement alkyle inférieur.

6. Procédé selon l'une quelconque des revendications 1 à 5, dans lequel Z du chloro-alkanoate (II) est un méthylène ou un méthylméthylène.

7. Procédé selon l'une quelconque des revendications 1 à 6, dans lequel le chloro-alkanoate (II) est le chloro-acétate d'éthyle.

8. Procédé selon l'une quelconque des revendications 1 à 7, dans lequel le chloro-alkanoate (II) est utilisé dans une proportion molaire de 1 à 5 fois par rapport à la tétrahydroquinazoline (I).

9. Procédé selon l'une quelconque des revendications 1 à 8, dans lequel l'iodure de métal alcalin est l'iodure de potassium ou l'iodure de sodium.

10. Procédé selon la revendication 2, qui est exécuté dans au moins un solvant choisi parmi un hydrocarbure aromatique, un halogénure d'hydrocarbure et un solvant d'éther.

11. Procédé selon la revendication 2, dans lequel le solvant est un hydrocarbure aromatique substitué par un halogène.

12. Procédé selon la revendication 1, dans lequel la réaction avec le chloro-alkanoate (II) est exécuté tout en séparant par distillation les composés générés qui présentent un point d'ébullition inférieur.
